# EUROPEAN PATENT SPECIFICATION

(11) **EP 0 993 447 B1**
(45) Date of publication and mention of the grant of the patent: **17.08.2005**
(21) Application number: 99912189.0
(22) Date of filing: 08.04.1999
(51) Int. Cl.: C07D 211/90

(54) **A PROCESS FOR THE PREPARATION OF AMLODIPINE BENZENESULPHONATE**
EIN VERFAHREN ZUR HERSTELLUNG VON AMLODIPIN BENZOLSULFONAT
PROCEDE DE PREPARATION D'AMLOPIDINE BENZENESULFONATE

(30) Priority: 09.04.1998 PL 32575798
(43) Date of publication of application: 19.04.2000
(73) Proprietor: Adamed SP. Z O.O., 05-152 Czosnow k/Warszawy (PL)
(72) Inventor: WLOSTOWSKI, Marek, PL-01-312 Warszawa (PL); WIECZOREK, Maciej;, PL-27-400 Ostrowiec Swietokrzyski (PL)
(74) Representative: HOFFMANN EITLE
(86) International application number: PCT/PL1999/000011
(87) International publication number: WO 1999/052873

(56) References cited:
- EP-A- 0 244 944

## Description

The present invention relates to a process for the preparation of amlodipine benzenesulphonate, i.e. 2-(2-aminoethoxymethyl)-4-(2-chlorophenyl)-3-ethoxycarbonyl-5-methoxycarbonyl-6-methyl-1,4-dihydropyridine monobenzenesulphonate of the formula I as presented on the annexed drawing.

Amlodipine is a modern medicament belonging to the group of calcium channel blockers. It has a significant selectivity against resistance arterioles and coronary arteries and specific pharmacokinetic properties: good bioavailability, long half-life, slow onset and decline of action onset as well as long-lasting pharmacological reaction, any substantial interactions with other medicaments being absent.

Due to these advantages amlodipine is utilized successfully in the treatment of arterial hypertension as a first choice therapeutic agent; it is also used successfully in the treatment of coronary disease, including Prinzmetal angina and other circulatory system diseases.

While amlodipine shows biological activity in its free base form, it is used in the pharmaceutical preparations as a salt with pharmacologically acceptable acids.

The European Patent Application EP 089,167 discloses a series of pharmaceutically acceptable amlodipine salts such as hydrochloride, hydrobromide, sulphate, phosphate, acetate, maleate, tartrate, citrate and others. Maleate is indicated as the most preferred salt.

The European Patent Application EP 0244944 discloses the process for the preparation of amlodipine benzenesulphonate, which comprises treating amiodipine as a free base with benzenesulphonic acid or alternatively with benzenesulphonic acid ammonium salt in an inert organic solvent. In the examples of realisation (Examples I and V) industrial methylated spirit is used as a solvent.

Amlodipine benzenesulphonate has been accepted for amlodipine administration both in the form of tablets and sterile aqueous solutions.

Amlodipine benzenesulphonate shows certain physical properties making it particularly destined for a pharmacologically acceptable amlodipine salt. It is much more stable than other salts both as a solid and a solution; it is relatively well soluble in water (4.6 mg/ml) but not hygroscopic. The pH of a saturated aqueous solution is about 6.6 being relatively close to the blood pH 7.4. Finally, due to its excellent mechanical properties can be easily compressed, forming tablets of a good quality without adhering to the punch of the tabletting machine, etc.

However, while amlodipine benzenesulphonatie excellently meets the requirements for a good pharmaceutical material, the known process for preparing thereof has some disadvantages.

The process for the preparation of amlodipine benzenesulphonate according to EP 0244 944 comprises reacting free base of amlodipine with benzenesulphonic acid. The process is performed in an alcohol and thus may cause some fire hazard due to alcohol inflammability. The additional disadvantage is due to the fact that the reaction utilizes the free benzenesulphonic acid, which is a caustic, corroding and noxious substance. Additionally, due to its high hygroscopicity the acid requires the special safeguards during transport and handling and in practice is used in the form of dense oily material containing about 90% of acid and about 10% of water.

The alternative process also presents some hazards. Although the dangerous benzenesulphonic acid has been replaced with its ammonium salt, thus eliminating hazards and drawbacks connected with the use of free acid, the formation of amlodipine benzenesulphonate is accompanied, however, with the evolution of gaseous ammonia which is toxic and dangerous and has to be additionally absorbed and deactivated. Of course, the fire hazard connected with an inflammable alcohol is still present.

The above-discussed hazards and difficulties are eliminated by the process for the preparation of amlodipine benzenesulphonate of the present invention.

According to the invention, a process for the preparation of amlodipine benzenesulphonate of Formula I, 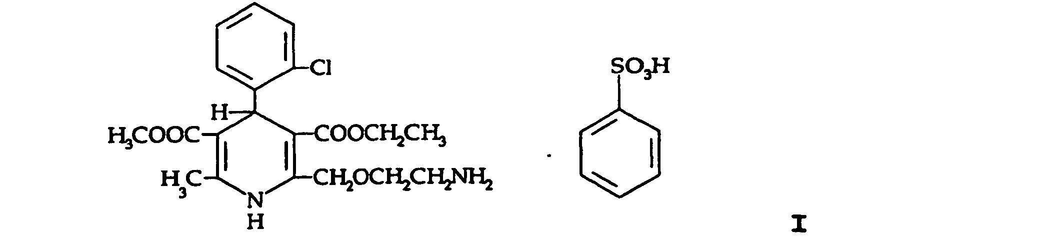 is characterized in that amlodipine salt with an inorganic or organic acid selected from acetate, formate, chloroacetate, hydrobromide, nitrate, hydrochloride and methanesulphonate is reacted with alkali metal benzenesulphonate in an aqueous medium or in a mixture water-alcohol C₁-C₂.

Preferably the amiodipine salt is hydrochloride, acetate or formate, especially hydrochloride.

Alkali metal benzenesulphonate comprises lithium, sodium and potassium benzenesulphsnate. Particularly preferred is sodium benzenesulphonate as an inexpensive, safe, stable and commercially available chemical product.

Preferred water-alcohol mixture is the mixture water-ethanol, comprising from 20 to 50% (v/v) of ethanol, especially 1:1 mixture.

The process of the invention may be realised by preparing a solution of amlodipine salt in water or a water-alcohol mixture, and adding, preferably at 5-40°C with vigorous stirring, a solution of sodium benzenesulphonate in water in a stoichiometric amount or preferably at a molar ratio of sodium benzenesulphonate/amlodipine salt being 1:1.15. The mixture is stirred for about 10-60 minutes, optionally warmed to 40°C and then cooled to 10°C. The resulting precipitate of amlodipine benzenesulphonate is filtered off, washed twice with water and dried. If the salt separates as an oil, it is necessary to add some amlodipine benzenesulphonate crystals to speed the crystallization process. The product thus obtained contains no contaminants. Alternatively, the process can be performed by adding solid sodium benzenesulphonate to the amlodipine salt. The reverse order of reagents addition, i.e. adding the amlodipine salt to the solution of sodium benzenesulphonate in water also results in a highly pure product.

The following non-limiting Examples are presented below to illustrate the invention:

### Example 1

To the water (150 ml) amlodipine hydrochloride (71.5 g) was added and the mixture was stirred for 15 minutes at 20°C. The solution of sodium benzenesulphonate (33.3 g) in 200 ml of water was added portionwise during 10 minutes. A small amount of amlodipine benzenesulphonate crystals as seeds for crystallization was added and the mixture was stirred for 40 minutes. It was then cooled to 10°C and the resulting precipitate filtered off. The precipitate was washed with distilled water (3x 100 ml) and dried. 80.0 g of amlodipine benzenesulphonate was obtained, mp=201°C. Yield: 88%.

### Example 2

To the solution of sodium benzenesulphonate (4 g) in water (20 ml) amlodipine formate (9.1 g) was added portionwise with stirring at 20°C. After addition had been completed, the mixture was stirred for 20 minutes, then cooled to 5°C and the product precipitate filtered off. The precipitate was washed with water (2 x 20 ml) and dried in vacuo. 18.8 g of amlodipine benzenesulphonate was obtained, mp=201°C. Yield: 90%.

### Example 3

To the solution of amlodipine hydrobromide (9.6 g) in water (25 ml) sodium benzenesulphonate (4 g) was added portionwise with vigorous-stirring. After addition had been completed, the mixture was stirred for 20 minutes, then cooled to 5°C, and following the procedure of Example 2 11.6 g of amlodipine benzenesulphonate was obtained, mp=201°C.

### Example 4

To the solution of sodium benzenesulphonate (4 g) in water (10 ml) amlodipine acetate (9.3 g) in 20 ml of the water-ethanol mixture (1:1) was added portionwise with stirring at 20°C. After addition had been completed, the mixture was stirred for 30 minutes, then cooled to 5°C, and several crystals of amlodipine benzenesulphonate and additionally 10 ml of water were added. Following the procedure of Example 2 amlodipine benzenesulphonate was obtained with the yield of 83%, mp=201°C.

### Example 5

Starting from amlodipine chloroacetate (10.6 g) and sodium benzenesulphonate (4 g) and following the procedure of Example 1, amlodipine benzenesulphonate was obtained with 89% yield, mp=201°C.

### Example 6

Starting from amlodipine methanesulphonate (10.6 g) and sodium benzenesulphonate (4 g) and following the procedure of Example 1, amlodipine benzenesulphonate was obtained with 81% yield, mp=201°C.

### Example 7

Starting from amlodipine nitrate (9.4 g) and sodium benzenesulphonate (4 g) and following the procedure of Example 1, amlodipine benzenesulphonate was obtained with 83% yield, mp=201°C.

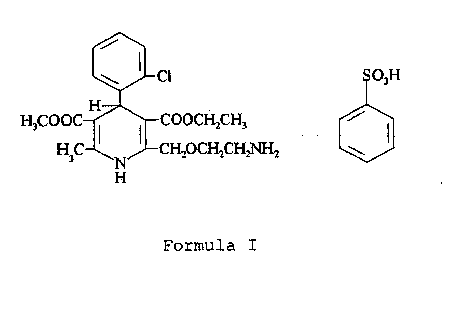

## Claims

1. A process for the preparation of amlodipine benzenesulphonate of the Formula I, 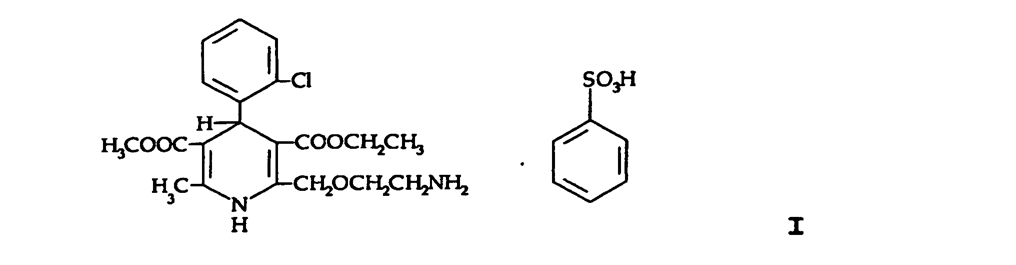 **characterised in that** a salt of amlodipine with an inorganic or organic acid selected from acetate, formate, chloroacetate, hydrobromide, nitrate, hydrochloride or methanesulphonate is reacted with alkali metal benzenesulphonate in an aqueous medium or in a mixture water-alcohol C₁-C₂.

2. The process according to claim 1, **characterised in that** the amlodipine salt is hydrochloride.

3. The process according to any one of claims 1-2, **characterised in that** the alkali metal benzenesulphonate is sodium benzenesulphonate.

4. The process according to any one of claims 1-3, **characterised in that** the reaction is performed in the water-ethanol mixture containing from 20 to 50% of ethanol, especially 1:1 mixture.

5. The process according to any one of claims 1-3, **characterised in that** the reaction is performed in the aqueous medium.

6. The process according to any one of claims 1-5, **characterised in that** the reaction is performed at the temperature 5-40°C.

## Patentansprüche

1. Verfahren zur Herstellung von Amlodipinbenzolsulfonat der Formel (I): 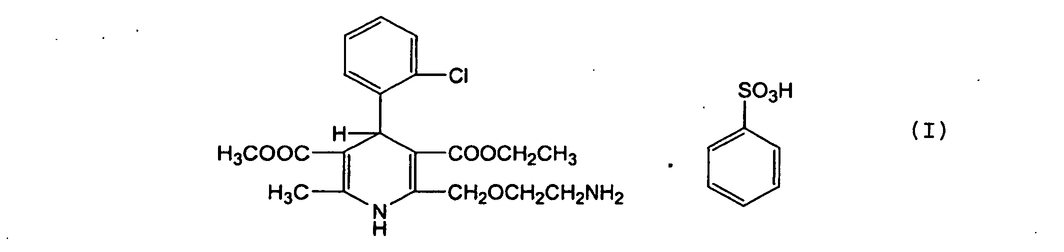 **dadurch gekennzeichnet, daß** ein Salz aus Amlodipin mit einer anorganischen oder organischen Säure, ausgewählt aus Acetat, Formiat, Chloracetat, Hydrobromid, Nitrat, Hydrochlorid oder Methansulfonat, mit Alkalimetallbenzolsulfonat in einem wäßrigen Medium oder in einer Wasser-C₁-C₂-Alkohol-Mischung umgesetzt wird.

2. Verfahren gemäß Anspruch 1, **dadurch gekennzeichnet, daß** das Amlodipinsalz ein Hydrochlorid ist.

3. Verfahren gemäß einem der Ansprüche 1 bis 2, **dadurch gekennzeichnet, daß** das Alkalimetallbenzolsulfonat Natriumbenzolsulfonat ist.

4. Verfahren gemäß einem der Ansprüche 1 bis 3, **dadurch gekennzeichnet, daß** die Reaktion in einer Wasser-Ethanol-Mischung durchgeführt wird, die 20 bis 50 % Ethanol enthält, insbesondere eine 1:1 Mischung.

5. Verfahren gemäß einem der Ansprüche 1 bis 3, **dadurch gekennzeichnet, daß** die Reaktion in dem wäßrigem Medium durchgeführt wird.

6. Verfahren gemäß einem der Ansprüche 1 bis 5, **dadurch gekennzeichnet, daß** die Reaktion bei der Temperatur von 5 bis 40°C durchgeführt wird.

## Revendications

1. Procédé pour la préparation de benzèneaulfonate d'amlodipine de la formule I, 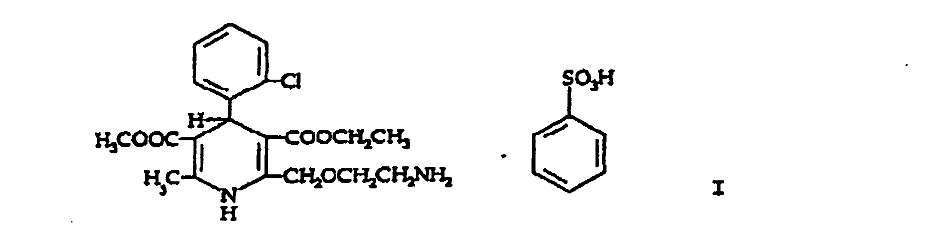 **caractérisé en ce qu'**un sel d'amlodipine avec un acide inorganique ou organique choisi parmi un acétate, un formiate, un chloroacétate, un bromhydrate, un nitrate, un chlorhydrate ou un méthanesulfonate est mis à réagir avec un benzèriesulfonate de métal alcalin dans un milieu aqueux ou dans un mélange eau-alcool en C₁-C₂.

2. Procédé selon la revendication 1, **caractérisé en ce que** le sel d'amlodipine est un chlorhydrate.

3. Procédé selon l'une quelconque des revendications 1-2, **caractérisé en ce que** le benzènesulfonate de métal alcalin est le benzènesulfonate de sodium.

4. Procédé selon l'une quelconque des revendications 1-3, **caractérisé en ce que** la réaction est effectuée dans le mélange eau-éthanol contenant de 20 à 50 % d'éthanol, plus particulièrement un mélange 1 : 1.

5. Procédé selon l'une quelconque des revendications 1-3, **caractérisé en ce que** la réaction est effectuée dans le milieu aqueux.

6. Procédé selon l'une quelconque des revendications 1-5, **caractérisé en ce que** la réaction est effectuée à la température de 5-40 °C.
